# EUROPEAN PATENT SPECIFICATION

(11) **EP 0 538 291 B1**
(45) Date of publication and mention of the grant of the patent: **14.09.1994**
(21) Application number: 91911981.8
(22) Date of filing: 25.06.1991
(51) Int. Cl.: C07C 271/20, C07C 259/06

(54) **INTERMEDIATE USED FOR THE PREPARATION OF DEFEROXAMINE**
ZWISCHENPRODUKT FÜR DIE HERSTELLUNG VON DEFEROXAMINE
INTERMEDIAIRE UTILISE POUR LA PREPARATION DE LA DEFEROXAMINE

(30) Priority: 06.07.1990 US 548717; 04.02.1991 US 649864
(43) Date of publication of application: 28.04.1993
(73) Proprietor: THE UPJOHN COMPANY, Kalamazoo, Michigan 49001 (US)
(72) Inventor: WUTS, Peter, G., M., Galesburg, MI 49053 (US)
(74) Representative: Perry, Robert Edward
(86) International application number: US9104339
(87) International publication number: WO9200957

(56) References cited:
- EP-A- 0 347 163
- US-A- 3 247 197

## Description

The present invention is directed toward a novel, key intermediate useful for the preparation of deferoxamine. Deferoxamine is well known in the art as a natural product which is a microbial iron chelator and was first isolated from Streptomyces pilosus which utilized it to obtain iron from the environment. Its synthesis and characterization were documented by Bickel (Helv. Chim. Acta., Vol.43. p. 2129) in 1960. Deferoxamine has various pharmaceutical uses such as the treatment of hemodialysis-induced aluminum accumulation in the brain and for iron overload conditions.

The synthesis of deferoxamine and its analogs has been described in various publications such as U.S. Patents 3,471,476 and 3,247,197 and European Patent Application 0 347 163 published 20 December 1989. Despite the various methods disclosed for the synthesis of deferoxamine new and more economical means for synthesis have been sought. The present invention discloses a key intermediate which can be prepared from readily available ingredients and using conventional chemistry. This provides a distinct advantage over previous methods for the synthesis of deferoxamine which have required the use sensitive chemical procedures or difficult to prepare intermediates.

### INFORMATION DISCLOSURE STATEMENT

Various synthesis methods for preparing deferoxamine are described in publications such as Bickel, Helv. Chim. Acta., **43** 2129 (1960); Helv. Chim. Acta., **45** 631 (1962); and R. J. Bergeron and J. J. Pegram, J. Org. Chem., **53** 3131 (1988).

U.S. Patents 3,118,823; 3,153,621; 3,158,552; 3,247,197 and 3,471,476 describe the general state of the art with respect to deferoxamine. The latter two deal with the chemical synthesis of deferoxamine. European Patent Application publication to the University of Florida publication number 0 347 163 also describes the chemistry of deferoxamine similar to that of Bergeron.

### SUMMARY OF THE INVENTION

In one aspect, the subject invention is an intermediate useful in the preparation of deferoxamine having Formula I

X-Ph-CH₂-O-C(O)-NH-(CH₂)₄-CH=NOR

wherein X is a C₁-C₄ alkyl, -O(C₁-C₄ alkyl), a halogen or hydrogen; R is X-Ph-CH₂- or hydrogen. In one preferred embodiment X and R are independently hydrogen or X is a methyl or methoxy group. In another preferred embodiment X and R are hydrogen. The intermediate has the advantage of being prepared in fewer and more efficient steps from readily available materials than conventional means for preparing deferoxamine.

In another aspect, the subject invention is the use of a compound of Formula I

X-Ph-CH₂-O-C(O)-NH-(CH₂)₄-CH=NOR

wherein X is a C₁-C₄ alkyl, -O(C₁-C₄ alkyl), a halogen or hydrogen, and R is X-Ph-CH₂- or hydrogen for preparing deferoxamine. The use comprises the steps of (a) reacting an oxime compound of Formula I to form a hydroxylamine; (b) reacting the hydroxylamine to form amides of formula 9: ZN(H)-(CH₂)₄-CHN(OH)Ac and formula 8: ZN(H)-(CH₂)₅-N(OH)-C(O)-(CH₂)₂-C(0)-OH (where Z is a protecting group, preferably, -C(O)OCH₂Ph); (c) reacting said compound of formula 8 with a chloroformate and base to form an anhydride; and (d) reacting said anhydride with an amine derived from formula 9 to yield a coupled product of Formula II: ZN(H)-(CH₂)₅-N(OH)-C(O)-(CH₂)₂C(O)-N(H)-(CH₂)₅-N(OH)Ac; (e) reacting an amine derived from Formula II with said anhydride to give a Z protected deferoxamine.

In yet another aspect, the present invention is directed toward a method for preparing an oxime intermediate of Formula I: X-Ph-CH₂-O-C(O)-NH-(CH₂)₄-CH=NOR wherein X is a C₁-C₄ alkyl, -O(C₁-C₄ alkyl), a halogen or hydrogen, R is X-Ph-CH₂- or hydrogen. The method comprises the steps of (a) reacting a compound structurally represented by formula 11: 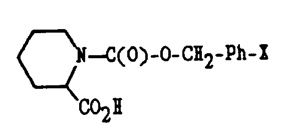
under electrolysis with a C₁₋₄ alcohol to form a compound structurally represented by formula 12: 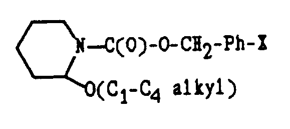
(b) treating said compound of formula 12 with a hydroxylamine hydrochloride in pyridine or a C₁₋₄ alkyl substituted pyridine. The method can include in step (b) the addition of a of C₁₋₄ alcohol, triethylamine or combination thereof. Preferably, formula 12 is converted to the oxime intermediate of Formula I by treatment with a hydroxylamine hydrochloride in pyridine and methyl alcohol.

### DETAILED DESCRIPTION OF THE INVENTION

The present invention is directed toward a key intermediate for the preparation of deferoxamine (also referred to as desferrioxamine). Desferrioxamine B (13 in Schemes, below) is an excellent chelator for iron (K_{f} = 10³⁰ M⁻¹) and is used to treat diseases such as thalassemias. The intermediate is shown as Formula I:

X-Ph-CH₂-O-C(O)-NH-(CH₂)₄-CH=NOR

wherein "X" is a C₁-C₄ alkyl, -O(C₁₋C₄ alkyl), a halogen (Cl, Br, F or I) or hydrogen any of which can be located at any of the positions on the phenyl ring (Ph); and where "R" is X-Ph-CH₂-or hydrogen.

A "C₁₋C₄ alkyl" is methyl, ethyl, propyl or butyl including isomeric forms thereof. Methyl is a preferred alkyl. A "C₁₋₄ alcohol" is methyl, ethyl, propyl or butyl alcohol including isomeric forms thereof. A preferred intermediate is where X and R are individually or simultaneously hydrogen which would be 1-carbobenzoxyamino-5-hydroxyliminopentane 6.

The subject intermediate is shown in Scheme I and II as oxime 6. It can be used to prepare the siderophore desferrioxamine 13. In the Schemes, Z is a protecting group well known to those skilled in the art of organic synthesis for preventing reaction at a particular site of a chemical compound. A preferred protecting group is -C(O)OCH₂Ph.

Desferrioxamine was prepared from the subject intermediate oxime 6 as outlined in the Scheme I. The oxime can also be prepared as outlined in scheme II. While the electrolysis reaction is not new the conversion of the amide to the oxime is new, i.e. reacting formula 8 with a chloroformate and base to form an anhydride. This sequence differs from that in Scheme I by replacement of the OH with an O-methyl group. Subsequent transformations of the oxime to the amides 8 and 9, where Z is a protecting group -C(O)OCH₂Ph, proceed via generally recognized methodology. The conversion of the hydroxamic acid 8 to the cyclic mixed anhydride 10 has been done with DCC or acetic anhydride as coupling agents. The conversion also proceeds with diisopropylcarbodiimide (DIC) or with a hindered acid chloride in the presence of base to form the cyclic mixed anhydride which is then used in the coupling steps. The use of DIC is an extension of the use of DCC. Another excellent means for forming the cyclic mixed anhydride is with isobutylchloroformate or the common variants of this reagent.

### Preparation of Intermediate, Oxime (6)

A solution of piperidine 60 ml, acetic acid 51 ml and water 50 ml was prepared. This solution was then added to a slurry of Ca(OCl)₂, 150 ml MTBE (methyl *t*-butyl ether) and 75 ml of water keeping the temperature between 0 and 10°C. When the addition is complete the solution was stirred for an additional 20 minutes. The chloramine was then isolated with MTBE (2 x 150 mL). The combined MTBE layers were concentrated to 110 ml and this solution was slowly added to a slurry of 45 g of KOH in 100 ml of MeOH keeping the temperature between 20 and 27°C. Potassium chloride precipitates from the mixture. The mixture is kept at room temperature overnight and then treated with 150 ml of saturated NaHCO₃. The BnOC(O)Cl is slowly added. The pH starts at about 14 and slowly drops as the BnOC(O)Cl is added. When the pH reaches 9, 50% NaOH is added to keep it between 9 and 10. When the addition is complete stir the solution for 1 or more hours and then extract with 3 x 100 mL of MTBE. The combined MTBE layers were dried over magnesium sulfate, filtered and concentrated to a pale yellow oil. The crude amide is taken up in 200 ml of MeOH and 100 ml of pyridine and treated with 44 g of hydroxylamine hydrochloride at reflux for 4 hours. MeOH was distilled from the mixture. After cooling the solution to ∼35°C, 400 ml of water was slowly added to knock out the oxime. The crystals were washed with water and MTBE and then dried with nitrogen to afford 69 g. of oxime, 48% yield. In general the yield of this reaction varies between about 45-65%. It should be noted that the initial conversion to the chloramine is not limited by the reaction described and that there are a number of other methods that can be used to accomplish this transformation.

The subject intermediate is prepared in efficient steps described above from readily available materials and represents an economical means for preparing deferoxamine. The final step as outlined above describes the use of hydroxylamine hydrochloride in pyridine and methyl alcohol however other embodiments can include the use of pyridine alone or its C₁₋₄ substituted forms thereof such as methyl pyridine or dimethyl pyridine. Typically, in order to avoid using large quantities of the pyridine or substituted pyridine a solvent such as a C₁₋₄ alcohol, triethylamine or combination thereof is used.

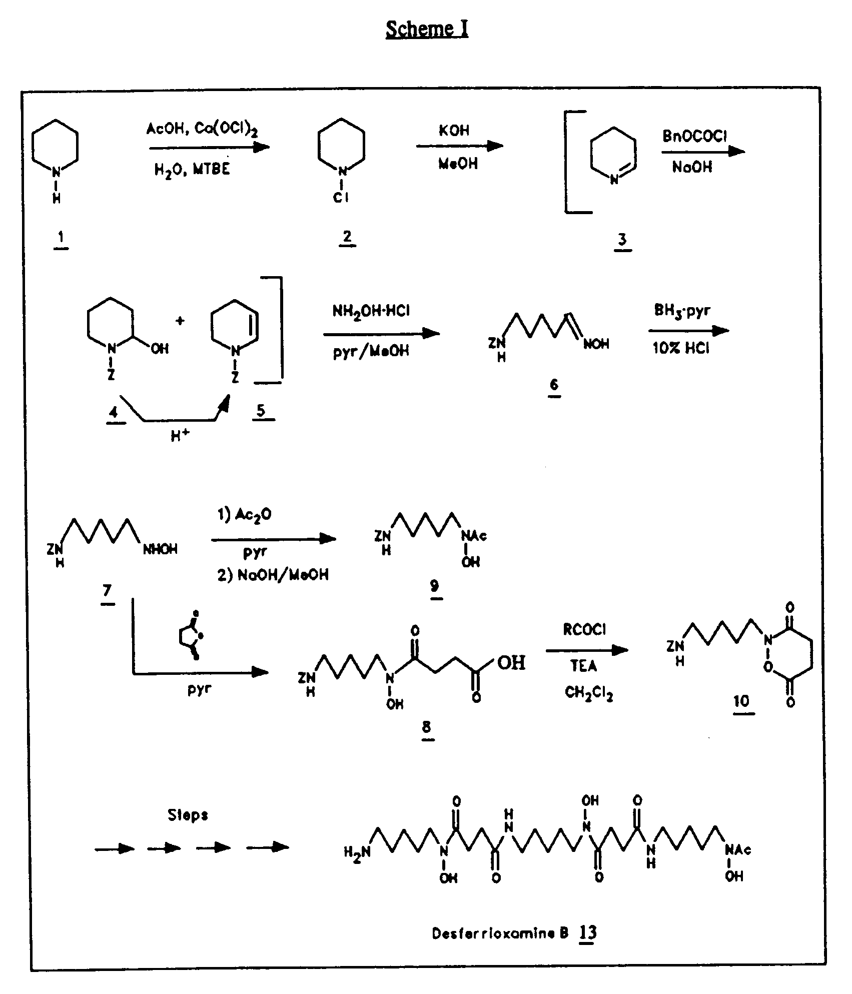

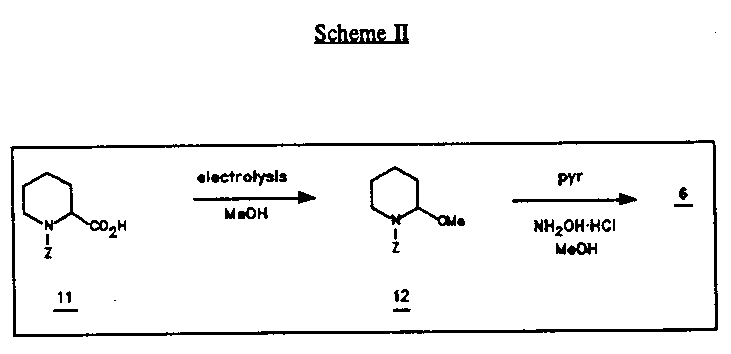

## Claims

1. A compound of Formula I
X-Ph-CH₂-O-C(O)-NH-(CH₂)₄-CH=NOR
wherein
X is a C₁₋C₄ alkyl, -O(C₁₋C₄ alkyl), a halogen or hydrogen;
R is X-Ph-CH₂- or hydrogen.

2. The compound of Claim 1 wherein R is hydrogen.

3. The compound of Claim 1 wherein X is hydrogen.

4. The compound of Claim 1 wherein X is a methyl or methoxy.

5. The compound of Claim 1 wherein X and R are hydrogen.

6. A method for preparing deferoxamine following steps comprising:
(a) reacting an oxime compound of Formula I to form a hydroxylamine wherein Formula I is
X-Ph-CH₂-O-C(O)-NH-(CH₂)₄-CH=NOR
wherein X is a C₁₋C₄ alkyl, -O(C₁-C₄ alkyl), a halogen or hydrogen, and R is X-Ph-CH₂- or hydrogen;
(b) reacting said hydroxylamine to form amides of
formula 9: ZN(H)-(CH₂)₄-CHN(OH)Ac and
formula 8: ZN(H)-(CH₂)₅-N(OH)-C(O)-(CH₂)₂-C(0)-OH,
where Z is a protecting group;
(c) reacting said compound of formula 8 with a chloroformate and base to form an anhydride;
(d) reacting said anhydride with an amine derived from formula 9 to yield a coupled product of Formula II: ZN(H)-(CH₂)₅-N(OH)-C(O)-(CH₂)₂C(O)-N(H)-(CH₂)₅-N(OH)Ac; and
(e) reacting an amine derived from Formula II with said anhydride to give deferoxamine.

7. The method of Claim 6 wherein the protecting group Z is -C(O)OCH₂Ph.

8. A method for preparing an oxime intermediate of Formula I
X-Ph-CH₂-O-C(O)-NH-(CH₂)₄-CH=NOR
wherein X is a C₁-C₄ alkyl, -O(C₁-C₄ alkyl), a halogen or hydrogen, R is X-Ph-CH₂- or hydrogen, comprising:
(a) reacting a compound structurally represented by formula 11 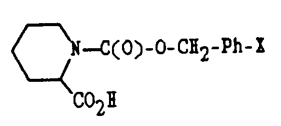 under electrolysis with a C₁-C₄ alcohol to form a compound structurally represented by formula 12 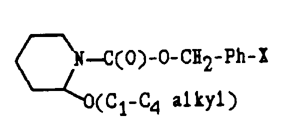
(b) treating said formula 12 with a hydroxylamine hydrochloride in pyridine or a C₁₋₄ alkyl substituted pyridine.

9. The method of Claim 8 wherein said step (b) includes the addition of a of C₁₋₄ alcohol, triethylamine or combination thereof.

10. The method of Claim 8 wherein said formula 12 is converted to said oxime intermediate of Formula I by treatment with a hydroxylamine hydrochloride in pyridine and methyl alcohol.

## Patentansprüche

1. Verbindung der Formel I
X-Ph-CH₂-O-C(O)-NH-(CH₂)₄-CH=NOR
worin bedeuten:
X ein C₁-C₄-Alkyl, -O(C₁-C₄-Alkyl), ein Halogen oder Wasserstoff und
R X-Ph-CH₂- oder Wasserstoff.

2. Die Verbindung nach Anspruch 1, worin R für Wasserstoff steht.

3. Die Verbindung nach Anspruch 1, worin X für Wasserstoff steht.

4. Die Verbindung nach Anspruch 1, worin X für Methyl oder Methoxy steht.

5. Die Verbindung nach Anspruch 1, worin X und R für Wasserstoff stehen.

6. Verfahren zur Herstellung von Deferoxamin nach folgenden Stufen:
(a) Umsetzung einer Oximverbindung der Formel I
X-Ph-CH₂-O-C(O)-NH-(CH₂)₄-CH=NOR
worin X für ein C₁-C₄-Alkyl, -O(C₁-C₄-Alkyl), ein Halogen oder Wasserstoff steht und R X-Ph-CH₂- oder Wasserstoff darstellt,
zu einem Hydroxylamin;
(b) Umsetzung des Hydroxylamins zur Bildung von Amiden
der Formel 9: ZN(H)-(CH₂)₄-CHN(OH)Ac und
der Formel 8: ZN(H)-(CH₂)₅-N(OH(-C(O)-(CH₂)₂-C(O)-OH,
worin Z (jeweils) für eine Schutzgruppe steht;
(c) Umsetzung der Verbindung der Formel 8 mit einem Chlorkohlensäureester und einer Base zur Bildung eines Anhydrids;
(d) Umsetzung des Anhydrids mit einem von Formel 9 herrührenden Amin zur Bildung eines Kupplungsprodukts der Formel II:
ZN(H)-(CH₂)₅-N(OH)-C(O)-(CH₂)₂C(O)-N(H)-(CH₂)₅-N(OH)Ac
und
(e) Umsetzung eines von Formel II herrührenden Amins mit dem Anhydrid zur Bildung von Deferoxamin.

7. Verfahren nach Anspruch 6, worin die Schutzgruppe Z -C(O)OCH₂Ph entspricht.

8. Verfahren zur Herstellung eines Oximzwischenprodukts der Formel I
X-Ph-CH₂-O-C(O)-NH-(CH₂)₄-CH=NOR,
worin bedeuten:
X ein C₁-C₄-Alkyl, -O(C₁-C₄-Alkyl), ein Halogen oder Wasserstoff und
R X-Ph-CH₂- oder Wasserstoff,
durch
(a) Umsetzung einer Verbindung der Strukturformel 11 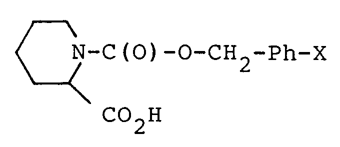 unter Elektrolyse mit einem C₁-C₄-Alkohol zur Bildung einer Verbindung der Strukturformel 12 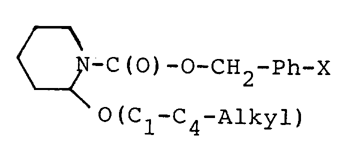 und
(b) Behandlung der Formel 12 mit einem Hydroxylaminhydrochlorid in Pyridin oder einem C₁₋₄-alkylsubstituierten Pyridin.

9. Verfahren nach Anspruch 8, wobei in Stufe (b) ein C₁₋₄-Alkohol, Triethylamin oder eine Kombination derselben zugesetzt wird.

10. Verfahren nach Anspruch 8, wobei die Formel 12 durch Behandeln mit einem Hydroxylaminhydrochlorid in Pyridin und Methanol in das Oximzwischenprodukt der Formel I überführt wird.

## Revendications

1. Composé de formule I
X-Ph-CH₂-O-C(O)-NH-(CH₂)₄-CH=NOR
dans laquelle
X représente un groupe alkyle en C₁ à C₄, -O-(alkyle en C₁ à C₄), un atome d'halogène ou un atome d'hydrogène ;
R représente un groupe X-Ph-CH₂ ou un atome d'hydrogène.

2. Composé suivant la revendication 1, dans lequel R représente l'hydrogène.

3. Composé suivant la revendication 1, dans lequel X représente l'hydrogène.

4. Composé suivant la revendication 1, dans lequel X représente un groupe méthyle ou méthoxy.

5. Composé suivant la revendication 1, dans lequel X et R représentent l'hydrogène.

6. Procédé de préparation de déféroxamine par les étapes comprenant :
(a) la réaction d'une oxime de formule I pour la formation d'une hydroxylamine, la formule I étant
X-Ph-CH₂-O-C(O)-NH-(CH₂)₄-CH=NOR
dans laquelle X représente un groupe alkyle en C₁ à C₄, -O-(alkyle en C₁ à C₄), un atome d'halogène ou un atome d'hydrogène ; et R représente un groupe X-Ph-CH₂ ou un atome d'hydrogène ;
(b) la réaction de ladite hydroxylamine pour former des amides de
formule 9 : ZN(H)-(CH₂)₄-CHN(OH)Ac, et
formule 8 : ZN(H)-(CH₂)₅N(OH)-C(O)-(CH₂)₂-C(O)-OH,
dans lesquelles Z représente un groupe protecteur ;
(c) la réaction dudit composé de formule 8 avec un chloroformiate et une base pour former un anhydride ;
(d) la réaction dudit anhydride avec une amine dérivée du composé de formule 9, ce qui donne un produit de couplage de formule II : ZN(H)-(CH₂)₅N(OH)-C(O)-(CH₂)₂-C(O)-N(H)-(CH₂)₅-N(OH)Ac ; et
(e) la réaction d'une amine dérivée de la formule II avec ledit anhydride, ce qui donne la déféroxamine.

7. Procédé suivant la revendication 6, dans lequel le groupe protecteur Z est un groupe -C(O)OCH₂Ph.

8. Procédé de préparation d'un intermédiaire du type oxime de formule I
X-Ph-CH₂-O-C(O)-NH-(CH₂)₄-CH=NOR
dans laquelle X représente un groupe alkyle en C₁ à C₄, -O-(alkyle en C₁ à C₄), un atome d'halogène ou un atome d'hydrogène, R représente un groupe X-Ph-CH₂- ou un atome d'hydrogène, comprenant :
(a) la réaction d'un composé représenté par la formule structurale 11 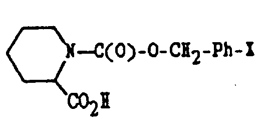 dans des conditions d'électrolyse avec un alcool en C₁ à C₄ pour la formation d'un composé représenté par la formule structurale 12 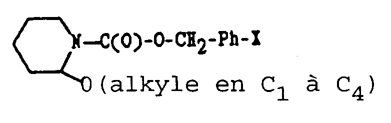
(b) le traitement dudit composé de formule 12 avec un chlorhydrate d'hydroxylamine dans la pyridine ou une pyridine à substituant alkyle en C₁ à C₄.

9. Procédé suivant la revendication 8, dans lequel l'étape (b) comprend l'addition d'un alcool en C₁ à C₄, de triéthylamine ou de leurs associations.

10. Procédé suivant la revendication 8, dans lequel le composé de formule 12 est transformé en l'intermédiaire du type oxime de formule I par traitement avec un chlorhydrate d'hydroxylamine dans la pyridine et l'alcool méthylique.
